Europäisches Patentamt
European Patent Office
Office européen des brevets

(11) Veröffentlichungsnummer: **0 298 478 A2**

# EUROPÄISCHE PATENTANMELDUNG

(21) Anmeldenummer: 88110871.6

(22) Anmeldetag: 07.07.88

(51) Int. Cl.4: C07C 45/00 , C07C 45/42 , C07C 49/16 , C07C 49/163 , C07C 49/167

Patentansprüche für folgenden Vertragsstaat: ES.

(30) Priorität: 10.07.87 CH 2643/87

(43) Veröffentlichungstag der Anmeldung: 11.01.89 Patentblatt 89/02

(84) Benannte Vertragsstaaten: AT BE CH DE ES FR GB IT LI NL

(71) Anmelder: CIBA-GEIGY AG
Klybeckstrasse 141
CH-4002 Basel(CH)

(72) Erfinder: Schaub, Bruno, Dr.
rue de Bellevie
CH-2822 Courroux(CH)

(74) Vertreter: Zumstein, Fritz, Dr. et al
Zumstein & Klingseisen Patentanwälte
Bräuhausstrasse 4
D-8000 München 2(DE)

## (54) Verfahren zur Herstellung von alpha-Fluorketonen.

(57) Die Umsetzung von α-Fluorcarbonsäureamiden von sekundären Aminen, die insgesamt mindestens 2 Heteroatome aus der Gruppe O, S und N enthalten, mit Organometallverbindungen ergibt in hohen Ausbeuten kristalline Salze von Halbaminalen, die zu den entsprechenden α-Fluorketonen hydrolisiert werden können. α-Fluorketone sind wertvolle Zwischenprodukte z.B. zur Herstellung von Fungiziden.

EP 0 298 478 A2

## Verfahren zur Herstellung von α-Fluorketonen

Die Erfindung betrifft ein Verfahren zur Herstellung von α-Fluorketonen durch Umsetzung von α-Fluorcarbonsäureamiden von sekundären Aminen, die insgesamt mindestens zwei Heteroatome aus der Gruppe O, S und N enthalten, mit Organometallverbindungen zu kristallinen Salzen von Halbaminalen und deren anschliessende Hydrolyse zu den entsprechenden α-Fluorketonen.

M. Nassal beschreibt im Liebigs Ann. Chem., S. 1510-1523 (1983) die Herstellung von Trifluor-p-methylacetophenon durch Umsetzung von N-Trifluoracetylpiperidin mit Lithiumtoluol und nachfolgender Hydrolyse. Die Ausbeuten sind bei dieser Reaktion gering.

Von D.A. Shaw et al. wird in Synthetic Communications, 15(14), S. 1291-1297 (1985) die Herstellung von Trifluor-o-methylacetophenon durch Umsetzung von N-Methyl-N-methoxy-trifluoracetamid mit o-Toluolmagnesiumbromid und nachfolgende Hydrolyse beschrieben. Es werden zwar verbesserte Ausbeuten erzielt. Hydroxamsäureester sind jedoch schwierig herstellbar und zudem teuer.

H. Staab et al. beschreiben in Liebigs Ann. Chem. 655, S. 90-94 (1962) die Umsetzung von Carbonsäureimidazoliden mit Phenyl- bzw. Alkylmagnesiumbromiden. Es werden relativ hohe Ausbeuten erzielt. Die Verwendung von α-Fluorcarbonsäureimidazoliden ist nicht erwähnt.

P.G. Gassmann et al. erwähnen im J. Org. Chem. 52: S. 2481-2490 (1987), dass bei der Umsetzung von Carbonsäureestern mit Lithiumpentafluorethyl dann Ketone gebildet werden, wenn bei -78 °C das Lithiumsalz eines Hemiketals entsteht, dass bei diesen Temperaturen hydrolisiert werden kann.

Y. Tominaga et al. beschreiben die Umsetzung von halogenfreien Thiocarbonsäuremorpholiden mit Lithiumphenyl oder Lithiumalkylen bei Raumtemperatur und nachfolgender Hydrolyse unter Desulfurierung zu Ketonen. Als Nebenprodukte entstehen Thioketone. Gignard- oder Organokupferreagenzien sind bei dieser Reaktion unwirksam. Die Bildung irgendwelcher Zwischenprodukte wird nicht erwähnt.

In den Publikationen ist nirgends erwähnt, dass isolierbare Zwischenprodukte gebildet werden. Es wurde gefunden, dass α-Fluorcarbonsäureamide von sekundären Aminen, die insgesamt mindestens zwei Heteroatome enthalten, mit Organometallverbindungen in hohen Ausbeuten zu den entsprechenden α-Fluorketonen reagieren. Bei der Reaktion entstehen überraschend kristalline Salze von Halbaminalen als Zwischenprodukte, die spontan aus dem Reaktionsgemisch ausfallen und einfach zu isolieren und zu reinigen sind. Nach deren Hydrolyse erhält man die gewünschten α-Fluorketone in hoher Reinheit. Es wurde ferner gefunden, dass die genannten α-Fluorcarbonsäureamide sehr reaktiv sind, und gegen Organometallreagenzien empfindliche Substituenten, z.B. Halogen oder Amidgruppen, im besagten α-Fluorcarbonsäureamid zugegen sein können, ohne dass die Ausbeuten dadurch wesentlich abnehmen.

Ein Gegenstand vorliegender Erfindung ist ein Verfahren zur Herstellung von α-fluorierten Ketonen der Formel I

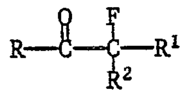

(I),

worin R lineares oder verzweigtes $C_1$-$C_{18}$-Alkyl-, $C_2$-$C_{18}$-Alkenyl-, $C_2$-$C_{18}$-Alkinyl; Cycloalkyl oder Cycloalkenyl mit 3 bis 8 Ring-C-Atomen, das mit $C_1$-$C_{12}$-Alkyl substituiert sein kann; über ein C-Atom an die CO-Gruppe gebundenes Heterocycloalkyl oder Heterocycloalkenyl mit 3 bis 7 Ringgliedern, das Heteroatome aus der Gruppe O, S und N enthält und das mit $C_1$-$C_{12}$-Alkyl substituiert sein kann; mit diesem Cycloalkyl, Cycloalkenyl, Heterocycloalkyl oder Heterocycloalkenyl substituiertes $C_1$-$C_{18}$-Alkyl, $C_2$-$C_{18}$-Alkenyl oder $C_2$-$C_{18}$-Alkinyl; $C_6$-$C_{14}$-Aryl, $C_7$-$C_{18}$-Alkaryl oder -Aralkyl, $C_8$-$C_{18}$-Alkaralkyl, -Aralkenyl oder -Aralkinyl, $C_9$-$C_{18}$-Alkaralkenyl oder -Alkaralkinyl; Heteroaryl mit 5 oder 6 Ringatomen, das Heteroatome aus der Gruppe O, N und S enthält und das mit $C_1$-$C_{12}$-Alkyl substituiert sein kann; oder mit diesem Heteroaryl substituiertes $C_1$-$C_{18}$-Alkyl, $C_2$-$C_{18}$-Alkenyl, $C_2$-$C_{18}$-Alkinyl darstellt; wobei dieser Rest R unsubstituiert oder mit Phenyl, Phenyl-$C_1$-$C_4$-alkyl, Phenoxy, Phenylthio, Benzyloxy, Benzylthio, $C_5$- oder $C_6$-Cycloalkyl, $C_5$- oder $C_6$-Cycloalkoxy, $C_5$- oder $C_6$-Cycloalkylthio, $C_1$-$C_{12}$-Alkoxy, $C_1$-$C_{12}$-Alkylthio, -Halogen, $-NO_2$, Sekundäramino, $C_1$-$C_{12}$-Acylamino, $C_1$-$C_{12}$-Aminocarbonyl, $R^3_3$ SiO-, wobei $R^3$ Phenyl, Benzyl oder $C_1$-$C_8$-Alkyl bedeutet, -O-$C_1$-$C_4$-Alkylen-O- oder -S-$C_1$-$C_4$-Alkylen-S-substituiert ist, $R^1$ und $R^2$ unabhängig voneinander für H, Halogen, $C_1$-$C_{18}$-Alkyl, Cycloalkyl oder Cycloalkenyl mit 3 bis 8 Ringkohlenstoffatomen, $C_6$-$C_{14}$-Aryl, $C_7$-$C_{18}$-Alkaryl oder -Aralkyl, $C_8$-$C_{18}$-Aralkenyl, -Aralkinyl oder -Alkaralkyl, $C_9$-$C_{18}$-Alkaralkenyl oder -Alkaralkinyl, $C_2$-$C_{18}$-Alkenyl, $C_2$-$C_{18}$-Alkinyl, Heterocycloalkyl oder Heterocycloalkenyl mit 3 bis 7 Ringgliedern, oder Heteroaryl mit 5 oder 6 Ringgliedern und Heteroatomen aus der Gruppe N, O und S; mit dem

genannten Cycloalkyl, Heterocycloalkyl, Heterocycloalkenyl oder Heteroaryl substituiertes $C_1$-$C_{18}$-Alkyl, $C_2$-$C_{18}$-Alkenyl oder $C_2$-$C_{18}$-Alkinyl, stehen, wobei $R^1$ und $R^2$ unsubstituiert oder wie für R definiert substituiert sein können, dadurch gekennzeichnet, dass man in einem inerten Lösungsmittel ein Carbonsäureamid der Formel II

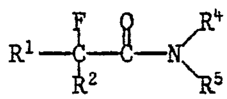 (II),

worin $R^1$ und $R^2$ die angegebene Bedeutung haben, $R^4$ $C_1$-$C_6$-Alkyl-X-$C_nH_{2n}$-ist worin X für -O- oder -N($C_1$-$C_{12}$-Alkyl)- und n für eine Zahl von 2 bis 4 stehen, $R^5$ $C_1$-$C_{12}$-Alkyl oder $C_1$-$C_{12}$-Alkyl-X-$C_nH_{2n}$-darstellt, oder $R^4$ und $R^5$ zusammen mit dem N-Atom, an das sie gebunden sind einen gesättigten oder ungesättigten Heterocyclus mit 5 bis 7 Ringgliedern darstellt, der insgesamt mindestens 2 Heteroatome aus der Gruppe O, S und $NR^6$ enthält und unsubstituiert oder mit $C_1$-$C_6$-Alkyl substituiert ist, und wobei $R^6$ für $C_1$-$C_6$-Alkyl, Phenyl, $C_1$-$C_{12}$-Alkylphenyl, Benzyl oder $C_1$-$C_{12}$-Alkylbenzyl steht, mit einer Verbindung der Formel III

$R\text{-}M^xY_{(x-1)}$ (III),

worin R die zuvor angegebene Bedeutung hat, Y für Halogenid, $M^x$ für ein Metallion aus der Gruppe $Li^{\oplus}$, $Na^{\oplus}$, $K^{\oplus}$, $Rb^{\oplus}$, $Mg^{2\oplus}$, $Zn^{2\oplus}$, $Cd^{2\oplus}$, $Cu^{\oplus}$, $Cu^{2\oplus}$, $Al^{3\oplus}$, $Ti^{4\oplus}$ und $Sn^{4\oplus}$ stehen und x die Valenz des Metallions bedeutet, zu einer Verbindung der Formel IV

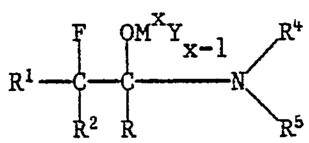 (IV),

worin R, $R^1$, $R^2$, $R^4$, $R^5$, $M^x$, Y und x die zuvor angegebene Bedeutung haben, umsetzt, und die Verbindung der Formel IV zu einer Verbindung der Formel I hydrolysiert.

R, $R^1$ und $R^2$ können lineares oder verzweigtes Alkyl mit bevorzugt 1 bis 12, besonders 1 bis 6 C-Atomen sein. Beispiele sind Methyl, Ethyl, n-oder i-Propyl, n-, i- oder t-Butyl, 1-, 2- oder 3-Pentyl, 1-, 2- oder 3-Hexyl, 1-, 2-, 3- oder 4-Heptyl, 1-, 2-, 3- oder 4-Octyl, Nonyl, Decyl, Undecyl, Dodecyl, Tetradecyl, Hexadecyl und Octadecyl.

Bei R, $R^1$ und $R^2$ kann es sich um lineares oder verzweigtes Alkenyl mit vorzugsweise 2 bis 12, besonders 2 bis 6 C-Atomen handeln. Die Doppelbindung kann endständig sein (Alkylvinyl) oder sich in der C-Kette befinden. Beispiele sind: Vinyl, Crotonyl, Allyl, But-1-en-1-yl, But-1-en-2-yl, But-1-en-3-yl, But-1-en-4-yl, But-2-en-2-yl, But-2-en-3-yl, But-2-en-4-yl, Pent-1-en-5-yl, Pent-1-en-1-yl, Pent-1-en-3-yl, Pent-2-en-2-yl, Pent-3-en-3-yl, Hex-1-en-6-yl, Hex-2-en-6-yl, Hex-3-en-6-yl, Hex-3-en-4-yl, Hex-3-en-5-yl, Heptenyl, Octenyl, Nonenyl, Decenyl, Undecenyl, Dodecenyl, Tetradecenyl, Hexadecenyl, Octadecenyl.

R, $R^1$ und $R^2$ kann lineares oder verzweigtes Alkinyl mit vorzugsweise 2 bis 12, besonders 2 bis 6 C-Atomen sein. Die Dreifachbindung kann endständig sein oder sich in der Kohlenstoffkette befinden. Beispiele sind Ethinyl, Propargyl, Prop-1-in-1-yl, But-1-in-1-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, Hex-1-in-1-yl oder -3-yl oder -4-yl oder -5-yl oder -6-yl, Hex-2-in-1-yl oder -4-yl oder -5-yl oder -6-yl, Hex-3-in-1-yl oder -2-yl, Heptinyl, Octinyl, Noninyl, Decinyl, Undecinyl, Dodecinyl, Tetradecinyl, Hexadecinyl, Octadecinyl.

Bei R, $R^1$ und $R^2$ kann es sich um gegebenenfalls mit vorzugsweise $C_1$-$C_4$-Alkyl substituiertes Cycloalkyl oder Cycloalkenyl mit vorzugsweise 4 bis 7, besonders 5 oder 6 Ring-C-Atomen handeln. Beispiele sind Cyclopropyl, Cyclobutyl, Cyclopentyl, Cyclohexyl, Cycloheptyl, Cyclooctyl, Cycloprop-1-en-1-yl, Cycloprop-1-en-3-yl, Cyclobut-1-en-1-yl, Cyclobut-1-en-3-yl, Cyclopent-1-en-1-yl, Cyclopent-1-en-3-yl, Cyclopent-1-en-4-yl, Cyclohex-1-en-1-yl, Cyclohex-1-en-3-yl, Cyclohex-1-en-4-yl, Cyclopentenyl, Cyclooctenyl.

R, $R^1$ und $R^2$ können Heterocycloalkyl, Heterocycloalkenyl oder Heteroaryl sein, die Heteroatome aus der Gruppe O, S und N enthalten. Bei dem N-Atom handelt es sich um ein teritäres N-Atom. Sofern das N-Atom im Ring als sekundäre Amingruppe vorliegt, ist dieses N-Atom z.B. $C_1$-$C_{12}$-, besonders $C_1$-$C_4$-alkyliert, phenyliert oder benzyliert. Es kann auch Schutzgruppen enthalten, z.B. $C_1$-$C_4$-Alkoxymethyl oder $R^3_3$ Si-Gruppen. Diese heterocyclischen Reste enthalten bevorzugt 1 bis 3, besonders 1 oder 2 gleiche oder verschiedene Heteroatome. Das Heterocycloalkyl bzw. -alkenyl enthält bevorzugt 5 oder 6 Ringglieder. Beispiele für Heterocyclen, von denen sich R, $R^1$ und $R^2$ ableiten können, sind (Schutzgruppen für

sekundäre N-Gruppen sind nicht erwähnt): Pyrrolidin, Tetrahydrofuran, Tetrahydrothiophen, Pyrrolin, Dihydrofuran, Dihydrothiophen, Indan, Dihydrocumaron, Dihydrobenzothiophen, Carbazol, Dibenzofuran, Dibenzothiophen, Pyrazolidin, Imidazolidin, Pyrazolin, Imidazolin, Benzimidazolidin. Oxazolidin, Oxazolin, Thiazolidin, Thiazolin, Isooxazolidin, Isooxazolin, Isothiazolidin, Isothiazolin, Benzoxazolidin, Benzisooxazolidin, Benzthiazolidin, 1,2,3- oder 1,2,4-Triazolidin, 1,2,3-oder 1,2,4-Triazolin, 1,2,3- oder 1,2,4-Oxazolidin oder -Oxazolin, Piperidin, Di- und Tetrahydropyridin, Dihydro- und Tetrahydropyran, Di-und Tetrahydrothiopyran, Piperazin, Dehydropiperazin, Morpholin, Thiomorpholin, 1,3- und 1,4-Dioxan, 1,4-Dithian, Azepan, 1,3-Dioxolan, 1,3-Dithiolan, Pyrrol, Indol, Imidazol, Benzimidazol, Furan, Thiophen, Benzofuran, Benzothiophen, Carbazol, Dibenzofuran, Dibenzothiophen, Oxazol, Isooxazol, Thiazol, Isothiazol, Benzoxazol, Benzothiazol, Pyridin, Pyrimidin, Pyrazin, Pyridazin, Chinolin, Isochinolin, Acridin, Chromen, Chroman, Pyran, Thiapyran, Phenazin, Phenoxazin, Phenolthiazin, Purin. $R^1$ und $R^2$ in der Bedeutung eines Heterocyclus sind vorzugsweise über ein C-Atom an die CF-Gruppe in Formel I gebunden. Sofern Cycloalkyl, Cycloalkenyl oder ein Heterocyclus mit Alkyl substituiert ist, handelt es sich vorzugsweise um $C_1$-$C_4$-Alkyl, besonders Methyl und Ethyl.

R, $R^1$ und $R^2$ kann mit gegebenenfalls mit $C_1$-$C_{12}$-, vorzugsweise $C_1$-$C_4$-Alkyl substituiertem Cycloalkyl, Cycloalkenyl, Hetrocycloalkyl, Heterocycloalkenyl, oder mit gegebenenfalls $C_1$-$C_{12}$-, vorzugsweise $C_1$-$C_4$-Alkyl substituiertem Heteroaryl substituiertes $C_1$-$C_{18}$-, vorzugsweise $C_1$-$C_{12}$-und besonders $C_1$-$C_6$-Alkyl, $C_2$-$C_{18}$-, vorzugsweise $C_2$-$C_{12}$- und besonders $C_2$-$C_6$-Alkenyl oder -Alkinyl sein. Die Doppelbindung bzw. Dreifachbindung kann endständig sein oder sich in der Kohlenstoffkette befinden. Beispiele für Alkyl-, Alkenyl- und Alkinyl sind zuvor erwähnt worden.

Bei R, $R^1$ und $R^2$ kann es sich um $C_6$-$C_{14}$-Aryl handeln. Beispiele sind Phenyl, Naphthyl, Anthracyl, Indenyl, Indanyl, Fluorenyl, Phenanthryl. Bevorzugt sind Phenyl, Naphthyl und Anthracyl.

R, $R^1$ und $R^2$ können Alkaryl oder Aralkyl mit vorzugsweise 7-14 C-Atomen, Aralkaryl, Aralkenyl, Aralkinyl mit vorzugsweise 8 bis 14 C-Atomen, oder Alkaralkenyl oder Alkaralkinyl mit vorzugsweise 9 bis 14 C-Atomen sein. Das Alkaryl, Alkaralkyl, -kenyl und -kinyl kann mehrere Alkylgruppen enthalten, vorzugsweise bis 3, die z.B. 1 bis 12, besonders 1-6 C-Atomen enthalten können. Das Aryl ist in diesen Resten bevorzugt Naphthyl und insbesondere Phenyl. Die Alkylengruppe im Aralkyl und Alkaralkyl enthält bevorzugt 1 bis 6, besonders 1 bis 3 C-Atome. Beispiele für solche Alkylengruppen sind Methylen, Ethylen, 1,2- oder 1,3-Propylen, 1,2-, 1,3-oder 1,4-Butylen, Pentylen, Hexylen, Heptylen, Octylen, Nonylen, Decylen. Das Alkenylen- und Alkinylen im Aralkenyl, Aralkinyl, Alkaralkenyl bzw. Alkaralkinyl enthält bevorzugt 2 bis 6, besonder 2 bis 3 C-Atome. Beispiele für solche Gruppen sind Vinylen, Ethinylen, Methylvinylen, Allylen, Propinylen, Butenylen und Butinylen.

$R^1$ und $R^2$ können zusätzlich H oder Halogen sein. Halogen ist bevorzugt F, Cl oder Br.

In einer bevorzugten Ausführungsform stellt R lineares oder verzweigtes $C_1$-$C_{12}$-Alkyl $C_2$-$C_{12}$-Alkenyl, $C_2$-$C_{12}$-Alkinyl, Cycloalkyl oder Cycloalkenyl mit 5 oder 6 Ring-C-Atomen, Heterocycloalkyl mit 5 oder 6 Ringgliedern, $C_6$-$C_{14}$-Aryl, $C_7$-$C_{12}$-Alkaryl oder -Aralkyl, $C_8$-$C_{14}$-Alkaralkyl, -Aralkenyl oder -Aralkinyl, $C_9$-$C_{16}$-Alkaralkenyl oder -Alkaralkinyl, oder Heteroaryl mit 5 oder 6 Ringgliedern dar.

In einer anderen bevorzugten Ausführungsform stehen $R^1$ und $R^2$ unabhängig voneinander für H, F, Cl, Br, $C_1$-$C_{12}$-Alkyl, Cycloalkyl mit 5 oder 6 Ring-C-Atomen, $C_6$-$C_{12}$-Aryl, $C_7$-$C_{14}$-Aralkyl oder -Alkaryl, $C_8$-$C_{14}$-Alkaralkyl, -Aralkenyl oder -Aralkinyl, $C_9$-$C_{14}$-Alkaralkenyl oder -Alkaralkinyl, $C_2$-$C_{12}$-Alkenyl, $C_2$-$C_{12}$-Alkinyl, Heterocycloalkyl, Heterocycloalkenyl oder Heteroaryl mit 5 oder 6 Ringgliedern und ein oder zwei Heteroatomen aus der Gruppe N, O und S.

In einer besonders bevorzugten Ausführungsform steht $R^1$ für H, F, Cl, Br, insbesondere für F, und $R^2$ hat die zuvor angegebene Bedeutung.

In einer weiteren bevorzugten Ausführungsform steht $R^1$ für F und $R^2$ stellt H, F, Cl, Br, $C_1$-$C_6$-Perfluoralkyl, $C_1$-$C_{12}$-Alkyl, $C_1$-$C_{12}$-Alkinyl, Cycloalkyl mit 5 oder 6 Ring-C-Atomen, $C_6$-$C_{12}$-Aryl, $C_7$-$C_{17}$-Aralkyl oder -Alkaryl oder $C_8$-$C_{14}$-Alkaralkyl dar.

Besonders bevorzugt ist $R^1$ F und $R^2$ bedeutet H, F, Cl, Br, $C_1$-$C_4$-Perfluoralkyl, $C_1$-$C_6$-Alkyl oder -Alkenyl.

R, $R^1$ und $R^2$ können unsubstituiert oder ein- oder mehrfach substituiert sein, z.B. mit 1 bis 4, vorzugsweise mit 1 bis 3 und besonders mit 1 oder 2 gleichen oder verschiedenen Substituenten. Bei Halogen als Substituent, insbesondere bei Fluor sind auch mehr als die bevorzugten 1 bis 4 Substituenten möglich, wie. z.B. in Polyfluor-, besonders Perfluoralkylresten oder Pentafluorphenyl.

Die Substituenten können ihrerseits unsubstituiert oder vorzugsweise einbis vierfach substituiert sein, besonders die aromatischen Substituenten. Beispiele für solche Substituenten sind $C_1$-$C_4$-Alkyl, $C_1$-$C_4$-Alkoxy, -$NO_2$, -F, -Cl, -Br, $C_2$-$C_8$-Sekundäramino, $C_1$-$C_4$-Acylamino, $C_1$-$C_4$-Aminocarbonyl, $R^3_3$ SiO-, wobei $R^3$ Phenyl, Benzyl oder $C_1$-$C_8$-Alkyl ist. Einige Beispiele für substituierte Substituenten sind Methoxyethoxy, Methoxycarbonylethoxy, Fluor- oder Chlorphenoxy, Toluyl, Methylbenzyl, Methoxyphenyl oder Methylcyclohexyl.

Bedeutet der Substituent Phenyl-$C_1$-$C_4$-alkyl, so kann es sich um Benzyl, 1-Phenyleth-1-yl, 1-Phenyleth-2-yl, 1-Phenyl- oder 2-Phenylprop-1-yl, -prop-2-yl oder -prop-3-yl, Phenylisopropyl, 1-Phenyl- oder 2-Phenylbut-1-yl, -but-2-yl, -but-3-yl oder -but-4-yl handeln. Cycloalkyl, Cycloalkoxy und Cycloalkylthio sind als Substituenten bevorzugt Cyclopentyl, Cyclopentoxy, Cyclopentylthio, Cyclohexyl, Cyclohexoxy und Cyclohexylthio.

Bedeutet der Substituent Alkoxy oder Alkylthio, so enthält er bevorzugt 1 bis 6, besonders 1 bis 4 C-Atome. Beispiele sind Methoxy, Methylthio, Ethoxy, Ethylthio, n- oder i-Propoxy, n- oder i-Propylthio, n- oder i-Butoxy, n- oder i-Butylthio. Als Halogen bedeutet der Substituent bevorzugt F, Cl oder Br und besonders F oder Cl.

Sekundäramino als Substituent kann der Formel $-NR^7R^8$ entsprechen, worin $R^7$ und $R^8$ unabhängig voneinander $C_1$-$C_8$-, bevorzugt $C_1$-$C_4$-Alkyl, besonders Methyl oder Ethyl, Cyclopentyl, Cyclohexyl, Phenyl oder Benzyl darstellen. $R^7$ und $R^8$ zusammen können Tri- oder Tetramethylen oder 3-Oxapentylen sein.

Ist der Substituent Acylamino oder Aminocarbonyl, so enthalten diese Reste bevorzugt 1 bis 6, besonders 1 bis 4 C-Atome. Die Aminogruppe im Acylamino kann mit $C_1$-$C_4$-Alkyl substituiert sein. Die Aminogruppe im Aminocarbonyl kann mit 1 oder 2 $C_1$-$C_4$-Alkyl substituiert sein. Beispiele sind Formylamino, Acetylamino, Propionylamino, Aminocarbonyl und Dimethylaminocarbonyl.

Beispiele für den Substituenten $R_3^3$ SiO- sind Trimethylsilyloxy, Dimethyl-t-butylsilyloxy und Dimethyl-(1,1,2,2-tetramethylethyl)-silyloxy.

Eine bevorzugte Untergruppe von Substituenten sind Phenyl, Benzyl, Phenoxy, Benzyloxy, $C_1$-$C_{12}$-Alkoxy, $C_1$-$C_{12}$-Alkylthio, F, Cl, Sekundäramino, oder $R_3^3$ SiO.

Eine bevorzugte Gruppe von substituierten Resten R ist mit 1 bis 5, besonders 2 bis 4 Fluoratomen und/oder Chloratomen substituiertes Phenyl, z.B. 2-Fluor-4-chlorphen-1-yl, 2-Fluor-4,6-dichlorphen-5-yl, 2-Chlorphen-1-yl, 4-Chlorphen-1-yl, 2,4-Dichlorphen-1-yl, 2,6-Dichlorphen-1-yl, 2,3-Dichlorphen-1-yl, 2,5-Dichlorphen-1-yl, 2,4,6-Trichlorphen-1-yl, 2,3,4-Trichlorphen-1-yl und 3,4,5-Trichlorphen-1-yl.

$R^5$ enthält als Alkyl bevorzugt 1 bis 6, besonders 1-4 C-Atome und stellt besonders Methyl oder Ethyl dar.

$R^4$ und $R^5$ können Alkyl-X-$C_nH_{2n}$- sein, worin das Alkyl bevorzugt 1-4 C-Atome enthält, und insbesondere Methyl oder Ethyl darstellt, n bevorzugt 2 oder 3 ist und die $C_nH_{2n}$-Gruppe 1,4-Butylen, 1,3- oder 1,2-Propylen und besonders Ethylen darstellt, und X- O- oder -N($C_1$-$C_{12}$-Alkyl)- ist, wobei die Alkylgruppe bevorzugt 1 bis 6, besonders 1 bis 4 C-Atome enthält und insbesondere Methyl oder Ethyl darstellt.

In einer bevorzugten Untergruppe ist $R^4$ $C_1$-$C_4$-Alkyl-X-$C_nH_{2n}$-, worin X -O-oder -N($C_1$-$C_4$-Alkyl)- und n 2 oder 3 sind und $R^5$ diese Bedeutung von $R^4$ hat oder $C_1$-$C_4$-Alkyl darstellt.

In einer bevorzugten Untergruppe ist $R^4$ $C_1$-$C_2$-Alkyl-X-$CH_2CH_2$-, worin X für -O- oder -N($C_1$-$C_2$-Alkyl)-steht, und $R^5$ diese Bedeutung von $R^4$ hat oder Methyl oder Ethyl darstellt.

Bilden $R^4$ und $R^5$ zusammen mit dem N-Atom einen Heterocyclus, so ist dieser vorzugsweise gesättigt, enthält vorzugsweise 5 und besonders 6 Ringglieder und enthält vorzugsweise insgesamt 2 oder 3, besonders 2 Heteroatome. Als Heteroatome sind -O- und $-NR^6$- bevorzugt. $R^6$ stellt bevorzugt $C_1$-$C_4$-Alkyl, Phenyl oder Benzyl, besonders Ethyl oder Methyl dar. Die Heteroatome befinden sich bevorzugt in 2-Stellung, besonders in 3-Stellung, bezogen auf das N-Atom. Der Heterocyclus ist bevorzugt unsubstituiert.

In einer bevorzugten Ausführungsform sind $R^4$ und $R^5$ zusammen mit dem N-Atom, an das sie gebunden sind, der Rest eines 5- oder 6-gliedrigen, gesättigten oder ungesättigten Heterocyclus mit 1 oder 2 weiteren Heteroatomen aus der Gruppe O, S und $NR^6$, wobei $R^6$ die zuvor angegebene Bedeutung hat.

Eine andere bevorzugte Ausführungsform ist dadurch gekennzeichnet, dass $R^4$ und $R^5$ zusammen mit dem N-Atom einen Rest der Formeln

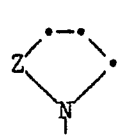 , 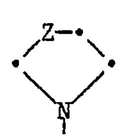 , 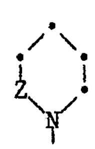 , 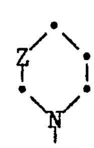 oder 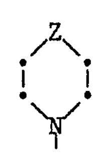

darstellen, worin Z für O, S oder $NR^6$ steht und $R^6$ $C_1$-$C_4$-Alkyl oder Phenyl bedeutet.

Eine besonders bevorzugte Ausführungsform ist dadurch gekennzeichnet, dass $R^4$ und $R^5$ zusammen mit dem N-Atom einen Rest der Formeln

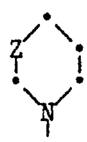 oder 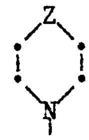

darstellen, worin Z O, S oder $NCH_3$ bedeutet.

Insbesondere stellt die Gruppe $-NR^4R^5$ Morpholinyl oder N-Methylpiperazinyl dar. Y in Formel III ist vorzugsweise Cl, Br oder I, besonders Cl oder Br.

Bei $M^x$ in Formel III handelt es sich bevorzugt um $Li^{\oplus}$, $Na^{\oplus}$, $Mg^{2\oplus}$, $Zn^{2\oplus}$ oder $Cd^{2\oplus}$ und besonders um $Li^{\oplus}$, $Mg^{2\oplus}$ oder $Zn^{2\oplus}$.

Die Verbindungen der Formel II sind bekannt oder können nach analogen Verfahren aus entprechenden Carbonsäureestern oder -chloriden und sekundären Aminen der Formel $HNR^4R^5$ hergestellt werden. Die Herstellung von Organometallverbindungen der Formel (III) erfolgt nach den üblichen Methoden.

Die Reaktionstemperatur beträgt zweckmässig -80 °C bis 60 °C, bevorzugt -30 °C bis 50 °C. Vorteilhaft wird ein inertes Lösungsmittels verwendet. Geignet sind inerte aprotische und bevorzugte polare Lösungsmittel. Beispiele sind aliphatische und cycoaliphatische Ether, wie zum Beispiel Diethylether, Di-n-butylether, Di-i-Butylether, Ethylenglycolmonobutylether, Tetrahydrofuran, 1,3-Dioxolan, 1,3- oder 1,4-Dioxan und N-Methylmorpholin.

Das Verfahren wird zweckmässig unter Schutzgas durchgeführt, z.B. Stickstoff oder Edelgasen, wie zum Beispiel Argon.

Im allgemeinen wird das Verfahren so durchgeführt, dass man die Organometallverbindung der Formel III vorlegt und die Verbindung der Formel II gegebenenfalls unter Kühlen zugibt. Hierbei bildet sich ein kristalliner Niederschlag der Verbindung der Formel IV. Zur Vervollständigung der Reaktion kann noch nachgerührt werden.

Der kristalline Niederschlag kann abfiltriert und durch Waschen z.B. mit einem bei der Reaktion verwendeten Lösungsmittel gereinigt werden. Zur Isolierung der Verbindung der Formel I wird die Verbindung der Formel IV hydrolisiert, entweder nach der Isolierung oder mit dem gesamten Reaktionsgemisch, zweckmässig mit verdünnten Mineralsäuren, z.B. Salz-, Schwefel- oder Phosphorsäure. Die Aufarbeitung des Hydrolysats erfolgt in bekannter Weise, z.B. Extraktion mit einem Lösungsmittel, Trocknen der Lösung, Abdestillieren des Lösungsmittels und fraktionierte Destillation oder Umkristallisation.

Die Verbindungen der Formel IV sind ein weiterer Gegenstand der Erfindung. Es gelten hierbei die für R, $R^1$, $R^2$, $R^4$, $R^5$, $M^x$ und Y erwähnten Bevorzugungen.

Die Verbindungen der Formel I sind wertvolle Zwischenprodukte, z.B. zur Herstellung von Fungiziden mit einem Triazolrest, wie sie in der EP-A-0 117 100, US-A-4 518 604 und 4 690 942 beschrieben sind. Ein weiterer Gegenstand der Erfindung sind Verbindungen der Formel Ia

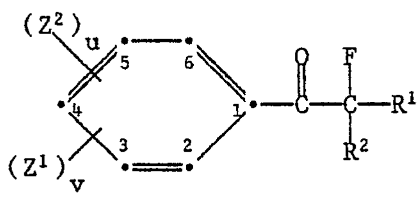 (Ia),

worin $R^1$ F oder Cl bedeutet und $R^2$ H ist oder unabhängig die Bedeutung von $R^1$ hat, $Z^1$ Cl und $Z^2$ F darstellen, v eine Zahl von 1 bis 5 und u 0 oder eine Zahl von 1 bis 4 sind, und die Summe u + v 2 bis 5 ist, ausgenommen 1-(2,4-Dichlorphenyl)-2,2,2-trifluorethanon.

Bei der Gruppe-$CFR^1R^2$ handelt es sich bevorzugt um $-CF_3$, $-CF_2H$, $-CF_2Cl$ und besonders $-CFCl_2$. Bevorzugt stellen v eine Zahl von 1 bis 3 und u 0, 1 oder 2 dar und die Summe u + v ist bevorzugt 2 oder 3. Insbesondere ist u = 0 und v = 2 oder 3.

Besonders bevorzugte Verbindungen der Formel Ia sind solche, worin $(Z^1_v)\!-$ und $(Z^2_u)\!-$ die Bedeutungen 2-Fluor-3-Chlor, 2,3-Dichlor, 2,4-Dichlor, 2-Chlor-4-Fluor, 2,6-Dichlor, 2,5-Dichlor, 2,4,6-Trichlor, 2,3,4-Trichlor oder 3,4,5-Trichlor haben.

Bei der Herstellung der Verbindungen der Formel Ia ist vorteilhaft, dass die Metallierung von Fluor- und/oder Chlorobenzolen zu entsprechenden Verbindungen der Formel III direkt mit z.B. Lithium-organischen Verbindungen erfolgen kann, und ein Halogen-Metall-Austausch unter Bildung von Metallhalogeniden vermieden werden kann.

Die Verbindungen der Formel I eignen sich auch zur Herstellung von Shift-Reagenzien für NMRspektrospkopische Bestimmungen von enantiomeren Verbindungen in deren Mischungen. Ein Beispiel ist (R)-(-)- bzw. (S)-(+)-1-(9-Anthryl)-2,2,2-trifluormethanol [siehe Aldrich Katalog, S. 176 (1986)]. Ferner können aus den Ketonen pestizide Oximcarbamate hergestellt werden (siehe US-PS 3,748,361). Es können auch Enzyminhibitoren aus den Verbindungen der Formel I hergestellt werden [siehe EP-A-0 130 679 und Tetrahedron Letters, Vol. 27, No. 37, S. 4437-4440 (1986)]. Auch die Herstellung pharmakologischer Wirkstoffe [Trifluor(aminoaryl)-ethanole] ist bekannt (siehe DE-OS 2 944 293).

Die nachfolgenden Beispiele erläutern die Erfindung näher.

Beispiel 1: Herstellung von α-Chlor-α,α-difluormethyl-isopropylketon

In einem 1,5 l Sulfierkolben werden unter Inertbedingungen 24,3 g (1,00 Mol) Magnesiumspäne mit 200 ml absolutem Diethylether (DEE) überschichtet. Dazu gibt man 25 g von insgesamt 123 g (1,00 Mol) Isopropylbromid und erwärmt das heterogene Gemisch so lange, bis die Grignardreagenz-Bildung einsetzt. Anschliessend wird das restliche Isopropylbromid, gelöst in 600 ml DEE so schnell zugetropft, dass der DEE am Rückfluss siedet.

Nach beendetem Zutropfen (45 Min.) wird die dunkle, noch einige Metallspäne enthaltende Grignardlösung 30 Min. bei 35 °C gerührt. Danach wird während 1 Std. unter Eis/Kochsalz-Kühlung eine Lösung von 160 g (0,80 Mol) α-Chlor-α,α-difluoressigsäuremorpholid in 200 ml DEE zugetropft, wobei sich bei der exothermen Reaktion sofort ein weisser kristalliner Niederschlag bildet. Nach dem Zutropfen des Amids wird die Suspension 30 Min. bei Raumtemperatur gerührt, dann wird das quantitativ ausgefallene leicht beige Zwischenprodukt (Schmelzpunkt 184-189 °C unter Zersetzung) abfiltriert, 1-mal mit 100 ml DEE nachgewaschen und auf ein Gemisch von 200 g Eis, 200 ml Salzsäure (37%ig) und 300 ml DEE gegossen. Die Wasserphase wird 3-mal mit je 100 ml DEE extrahiert. Die organischen Lösungen werden je einmal mit 100 ml 2N HCl und 100 ml $H_2O$ gewaschen und über $Na_2SO_4$ getrocknet. Der DEE wird über eine 30 cm Spaltrohrkolonne bei Normaldruck abdestilliert und der Rückstand fraktionniert destilliert. Beim Siedepunkt (Sdp.) 90-92 °C/975 mbar gehen 82,7 g (66 %) des Produktes als farblose Flüssigkeit über. Weitere Daten sind in Tabelle 1 angegeben.

Beispiel 2: Herstellung von α-Chlor-α,α-difluoracetophenon

In einem 350 ml Sulfierkolben werden 125 ml (120 mmol) Phenyllithium (0,96 molar) in DEE (hergestellt aus Brombenzol und Li in DEE; 1 Aequivalent LiBr-enthaltend) vorgelegt und mit 80 ml absolutem Pentan vermischt. Bei -30 °C ($CO_2$/$CCl_4$-Bad) wird innerhalb von 20 Min. eine Lösung von 21,3 g (100 mmol) α-Chlor-α,α-difluoracetylpiperazid in 30 ml DEE zugetropft, wobei die Temperatur auf -15 °C steigt und sich ein weisser, kristalliner Niederschlag bildet. Nach beendetem Zutropfen tauscht man das $CO_2$/$CCl_4$-Bad gegen ein Eisbad aus und lässt die Suspension 30 Min. bei 0 °C rühren. Danach wird das Zwischenprodukt (Schmelzpunkt 275-280 °C unter Zersetzung) rasch abfiltriert, einmal mit 60 ml DEE/Pentan (1:1) gewaschen und auf ein Gemisch von 50 g Eis, 50 ml Salzsäure (37%ig) und 100 ml DEE gegossen. Die wässrige Phase wird 3-mal mit je 50 ml DEE extrahiert. Die organischen Phasen werden je 1-mal mit 30 ml 2N HCl und $H_2O$ gewaschen, über $Na_2SO_4$ getrocknet und im Vakuumrotationsverdampfer eingeengt. Die Destillation des Rückstandes ergibt 15,4 g (81 %) Chlor-difluoracetophenon. Weitere Daten sind in Tabelle 1 angegeben.

Beispiele 3-14:

Es wird wie im Beispiel 2 verfahren. Die Produkte und Daten sind in Tabelle 2 angegeben.

Tabelle 1

| Beispiel Nr. | Keton | Ausbeute | Sdp. [°C] | $^{13}$C-NMR ($CDCl_3$)<br>δ >C=O u. δ $C-F_n$ [ppm]<br>(JC-CF u. JC-F [Hz]) |
|---|---|---|---|---|
| 1 | 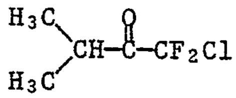 | 66 % | 90-92<br>975 mbar | 196,0 (t; $J_{C-CF}$ 28,2)<br>120,2 (t; $J_{C-F}$ 306,9) |
| 2 | 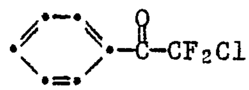 | 81 % | 114-116<br>130 mbar | 181,0 (t; $J_{C-CF}$ 28,7)<br>120,1 (t; $J_{C-F}$ 304,9) |
| 3 | 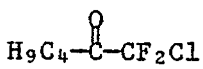 | 57 % | 144-145<br>988 mbar | 191,7 (t; $J_{C-CF}$ 28,9)<br>119,8 (t; $J_{C-F}$ 305,5) |
| 4 | 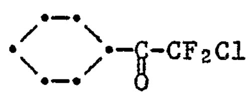 | 68 % | 116-117<br>130 mbar | 194,2 (t; $J_{C-CF}$ 30,0)<br>119,9 (t; $J_{C-F}$ 306,9) |
| 5 | 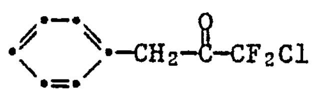 | 82 % | 118-119<br>65 mbar | 188,9 (t; $J_{C-CF}$ 29,3)<br>119,8 (t; $J_{C-F}$ 305,9) |
| 6 | 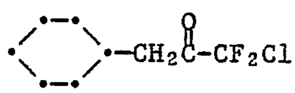 | 72 % | 70-72<br>23 mbar | 190,9 (t; $J_{C-CF}$ 28,9)<br>119,8 (t; $J_{C-F}$ 305,9) |
| 7 | 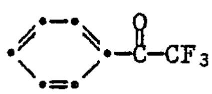 | 86 % | 88-89<br>130 mbar | 180,2 (q; $J_{C-CF}$ 34,9)<br>116,7 (q; $J_{C-F}$ 290,8) |
| 8 | 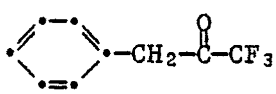 | 82 % | 97-98<br>130 mbar | 188,8 (q; $J_{C-CF}$ 35,3)<br>115,8 (q; $J_{C-F}$ 291,9) |
| 9 | 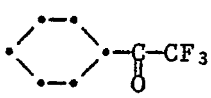 | 76 % | 84-86<br>130 mbar | 194,1 (q; $J_{C-CF}$ 33,1)<br>115,9 (q; $J_{C-F}$ 292,8) |
| 10 | 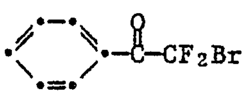 | 57 % | 124-125<br>130 mbar | 181,2 (t; $J_{C-CF}$ 25,7)<br>113,4 (t; $J_{C-F}$ 318,0) |
| 11 | 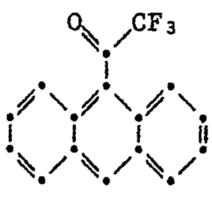 | 74 % | a)<br>83-85 | 191,1 (q; $J_{C-CF}$ 37,3)<br>116,0 (q; $J_{CF}$ 292,7) |

Tabelle 1 (Fortsetzung)

| Beispiel Nr. | Keton | Ausbeute | Sdp. [°C] | $^{13}$C-NMR ($CDCl_3$) δ >C=O u. δ $C-F_n$ [ppm] (JC-CF u. JC-F [Hz]) |
|---|---|---|---|---|
| 12 | 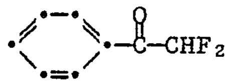 | 64 % | 86-88<br>23 mbar | 187,5 (t; $J_{C-CF}$ 25,1)<br>111,0 (t; $J_{C-F}$ 252,6) |
| 13 | 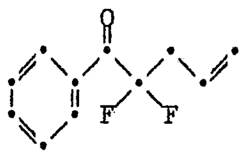 | 68 % | 101-103<br>21 mbar | 188,9 (t; $J_{C-CF}$ 31,1)<br>118,6 (t; $J_{C-F}$ 253,6) |
| 14[b)] | 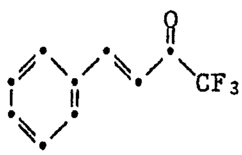 | 63 % | 90-93<br>23 mbar | 179,8 (q; $J_{C-CF}$ 35,2)<br>117,4 (q; $J_{C-CF}$ 290,4) |

a) Schmelzpunkt

b) trans-Olefin; $^1$H-NMR: 7,97 ppm (1H d; $J_{HH}$=15,5);
7,4 (5H; m)
7,02 (1H; d; $J_{HH}$=15,5).

Beispiel 15:

Es wird wie in den Beispielen 1 (Verwendung von Morpholid) oder 2 (Verwendungen von N-Methylpiperazid) verfahren und die Ausbeuten mittels Gaschromatographie in Gegenwart eines inneren Standards bestimmt. Es werden verschiedene Grignardreagenzien verwendet. Die Ausbeuten sind in Tabelle 2 angegeben.

Tabelle 2

| 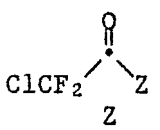 | 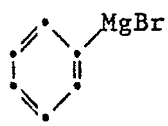 | 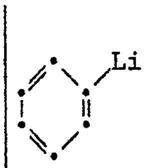 | 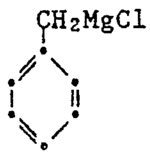 | 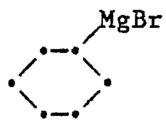 | 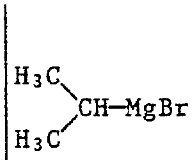 |
|---|---|---|---|---|---|
| 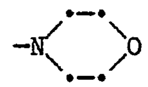 | 91 % | 88 % | 88 % | 76 % | 76 % |
| 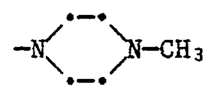 | 92 % | 87 % | 86 % | 76 % | 77 % |

Beispiel 16: Herstellung von 2-Chlor-α,α-difluormethyl-(2,4-dichlorphenyl)keton

In einem 1,5 l Sulfierkolben werden unter Inertbedingungen 140 ml (220 mmol) n-Butyllithium (1,58 M in Hexan) vorgelegt und unter $CO_2$/Ethanol-Kühlung während 15 Min. mit einer Lösung von 25,6 g (220 mmol) N,N,N',N'-Tetramethylethylendiamin (TMEDA) in 30 ml Diethylether (DEE) versetzt, wobei sich der n-Butyllithium/TMEDA-Komplex als weisser, voluminöser Niederschlag absetzt. Das Reaktionsgemisch wird kurzzeitig auf 0° C erwärmt. Zu der nun klaren, gelben Lösung werden innerhalb von 30 Min. 45.2 g (200 mmol) 2,4-Dichlorbrombenzol, gelöst in 150 ml DEE, bei -70° C zugetropft. Die weisse Suspension wird 30 Min. bei -70° C gerührt, dann bei derselben Temperatur innerhalb von 20 Min. mit einer Lösung von 42,6 g (200 mmol) α-Chlor-α,α-difluoracetyl-N-methylpiperazid in 30 ml DEE versetzt. Nach einstündigem Rühren bei -60° C wird die beige Suspension bei -10° C mit 200 ml 2N HCl hydrolisiert. Die wässrige Phase wird 3-mal mit je 100ml DEE extrahiert. Die organischen Lösungen werden je 1-mal mit 50 ml 2N HCl und $H_2O$ gewaschen, über $Na_2SO_4$ getrocknet und im Vakuumrotationsverdampfer (VR) eingeengt. Bei der Destillation des Rückstandes gehen beim Siedepunkt 122-123° C/35 mbar 39,0 g (75 %) des Produktes als farbloses Oel über.

$^1H$-NMR ($CDCl_3$; 300 MHz): 7,64 (d; J 8,4; 1H); 7,45 (d; J 2,0; 1H); 7,32 (d x d; J 8,4; 2,0; 1H).

$^{13}C$-NMR ($CDCl_3$; 75,4 MHz; B.B.-entkoppelt): 181,4 (t; $J_{C\text{-}CF}$31,0; C=O); 139,5 (-C⩽); 134,8 (-C⩽); 131,2 (CH); 130,5 (CH); 129,0 (-C⩽ ), 127,0 (CH); 119,7 (t; $J_{CF}$ 305,4; $CF_2Cl$).

Beispiel 17: Herstellung von α,α-Difluormethyl-(2,4-dichlorphenyl)keton

22,5 g (100 mmol) 2,4-Dichlorbrombenzol in 90 ml DEE werden wie in Beispiel 16 beschrieben zu 72 ml (110 mmol) n-Butyllithium (1,53 M in Hexan) und 12,8 g (110 mmol) TMEDA getropft. Nach der Zugabe von 17,9 g (100 mmol) α,α-Difluoracetyl-N-methylpiperazid in 15 ml DEE bei -70° C wird die beige Suspension 1 Std. bei -60° C gerührt, dann innerhalb von 30 Min. auf -10° C aufgetaut und mit 2N HCl hydrolisiert. Man erhält nach der Aufarbeitung und Destillation 18,0 g (80 %) eines farblosen Oels vom Sdp. 111-113° C/30 mbar.

$^1H$-NMR ($CDCl_3$; 300 MHz): 7,68 (d; J 8,5; 1H); 7,53 (d; J 2,0; 1H); 7,40 (d x d; J 8,5; 2,0; 1H); 6,38 (t; $J_{CF}$ 53,2; 1H).

$^{13}C$-NMR ($CDCl_3$; 75,4 MHz): 188,6 (t; $J_{C\text{-}CF}$ 26,3; C=O); 139,8 (-C⩽ ); 133,9 (-C⩽ ); 131,7 (CH); 131,0 (CH); 127,5 (CH); 126,8 (-C⩽ ); 109,7 (t; $J_{C\text{-}F}$ 252,6; $CHF_2$).

Beispiel 18: Herstellung von α,α,α-Trifluormethyl-(2,4-dichlorphenyl)keton

22,5 g (100 mmol) 2,4-Dichlorbrombenzol in 90 ml DEE werden wie in Beispiel 16 beschrieben zu 72 ml (110 mmol) n-Butyllithium (1,53 M in Hexan) und 12,8 g (110 mmol) TMEDA getropft. Nach der Zugabe von 18,4 g (100 mmol) α,α,α-Trifluoracetylmorpholid in 20 ml DEE bei -70 °C wird die weisse Suspension 1 Std. bei -60 °C gerührt, dann innerhalb von 30 Min. auf -10 °C aufgetaut und mit 2N HCl hydrolisiert. Man erhält nach der Aufarbeitung und Destillation 19,9 g (82 %) eines farblosen Oels vom Sdp. 108-111 °C/35 mbar.

$^1$H-NMR ($CDCl_3$; 300 MHz): 7,69 (d; J 8,2; 1H); 7,57 (d; J 2,0; 1H); 7,38 (d x d; J 8,2; 2,0).
$^{13}$C-NMR ($CDCl_3$; 75,4 MHz): 180,3 (q; $J_{C-CF}$ 37,1; C=O); 139,3 (-C< ); 134,6 (-C< ); 130,9 (CH); 130,4 (CH); 128,8 (-C< ); 127,0 (CH); 116,2 (q; $J_{CF}$ 291,4; $CF_3$).

Beispiel 19: Herstellung von α-Chlor-α,α-difluormethyl-(2,6-dichlorphenyl)keton

In einem 1,5 l Sulfierkolben werden unter Inertbedingungen 45,5 g (250 mmol) Dicyclohexylamin in 200 ml Tetrahydrofuran (THF) gelöst und innerhalb von 20 Min. mit 162 ml Butyllithium (1,55 M in Hexan) bei -70 °C versetzt. Der weisse, voluminöse Niederschlag wird 15 Min. bei -60 °C gerührt, dann kurzzeitig auf -20 °C erwärmt. Anschliessend tropft man innerhalb von 30 Min. bei -70 °C eine Lösung von 32,5 g (220 mmol) 1,3-Dichlorbenzol in 100 ml THF zu und rührt danach die hellgelbe Suspension 45 Min. bei -50 °C. Das Reaktionsgemisch wird während 10 Min. bei -50 °C mit 47,9 (240 mmol) α-Chlor-α,α-difluoracetylmorpholid, gelöst in 50 ml THF, versetzt und 30 Min. bei -50 °C gerührt. Anschliessend lässt man die Temperatur innerhalb von 30 Min. auf 0 °C ansteigen und hydrolisiert die weisse Suspension mit 150 ml 5N HCl. Nach dem Abfiltrieren des schlecht löslichen Dicyclohexylamin-hydrochlorid über eine Schicht Hyflo wird die wässerige Phase 3-mal mit je 100 ml DEE extrahiert. Die etherischen Phasen werden je 1-mal mit 50 ml 1N HCl und 50 ml $H_2O$ gewaschen, über $MgSO_4$ getrocknet und im VR eingeengt. Bei der Destillation des Rückstandes gehen beim Sdp. 118-119 °C/20 mbar 43,4 g (76 %) als farbloses Oel über.

$^{13}$C-NMR ($CDCl_3$; 75,4 MHz): 184,4 (t; $J_{C-CF}$ 34,7); 133,0 (-C< ); 132,5 (CH); 132,0 (2 x -C< ); 128,2 (2 x CH); 119,5 (t; $J_{CF}$ 304,8; $CF_2Cl$).
$^1$H-NMR ($CDCl_3$; 300 MHz): 7,41 (s; 3H).

Beispiel 20: Herstellung von α-Chlor-α,α-difluormethyl-(2,3-dichlorphenyl)keton

In einem 2 l 3-Halskolben werden 210 ml (330 mmol) n-Butyllithium (1,58 M in Hexan) vorgelegt und im Oelpumpenvakuum auf 1/3 seines Volumens eisngeengt. Danach wird die aufkonzentrierte n-Butyllithiumlösung auf -75 °C abgekühlt und mit 250 ml vorgekühltem THF vermischt. Bei derselben Temperatur werden 60,0 g (330 mmol) Dicyclohexylamin, gelöst in 100 ml THF während 10 Min. zugetropft. Danach wird die weisse Suspension kurzzeitig auf -30 °C erwärmt, dann innerhalb von 30 Min. bei -75 °C mit einer Lösung von 44,1 g (300 mmol) 1,2-Dichlorbenzol in 100 ml THF und gleich anschliessend mit 54,0 g (300 mmol) Hexamethylphosphorsäuretriamid versetzt. Man hält die intensiv rot gefärbte Lösung 30 Min. bei -70 °C und tropft danach 64,0 g (320 mmol) α-Chlor-α,α-difluoracetylmorpholid, gelöst in 200 ml THF, bei -70 °C zu. Die orange Suspension wird 1 Std. bei -70 °C gerührt, dann innerhalb von 30 Min. auf 0 °C erwärmt und unter $CO_2$/Ethanol-Kühlung mit 200 ml 5N HCl hydrolisiert. Das Gemisch wird über Hyflo filtriert. Die wässerige Phase wird 3-mal mit je 200 ml DEE gewaschen. Die Etherlösungen werden 1-mal mit 100 ml 2N HCl, dann 3-mal mit je 100 ml $H_2O$ gewaschen, über $MgSO_4$ getrocknet und im VR eingeengt. Der Rückstand, bestehend aus Produkt und nicht umgesetzten 1,2-Dichlorbenzol, wird einer fraktionierten Destillation unterworfen. Beim Siedepunkt 112-115 °C/20 mbar gehen 46,0 g (59 %) des Produktes als farbloses Oel über.

$^1$H-NMR ($CDCl_3$; 300 MHz): 7,68 (d x d; J 8,0; 1,5; 1H); 7,53 (d x q; J 8,0; 1,5; 1H); 7,35 (t; J 8,0).
$^{13}$C-NMR ($CDCl_3$; 75,1 MHz): 182,7 (t; $J_{C-CF}$ 31,7; C=O); 134,9 (-C< ); 133,9 (CH); 133,6 (-C< ); 128,2 (-C< ); 127,5 (CH); 127,0 (CH); 119,7 (t; $J_{CF}$ 305,4, $CF_2Cl$).

Beispiel 21: Herstellung von α-Chlor-α,α-difluormethyl-(2,5-dichlorphenyl)-keton

14,7 g (100 mmol) 1,4-Dichlorbenzol, gelöst in 50 ml THF, werden wie in Beispiel 20 bei -75 °C zu einer Suspension von 110 mmol Dicyclohexyllithiumamid [aus 20,0 g (110 mmol) Dicyclohexylamin und 71 ml (110 mmol) n-Butyllithium (1,55 M in Hexan; letzteres wird durch THF ersetzt)] in 120 ml THF getropft. Nach der Zugabe von 12,8 g (110 mmol) TMEDA wird die Suspension 1 Std. bei -60 °C gerührt, dann für kurze Zeit auf -20 °C erwärmt, wobei bei -30 °C eine exotherme Reaktion einsetzt. Anschliessend wurden 23,4 g (110 mmol) α-Chlor-α,α-difluoracetyl-N-methylpiperazid bei -60 °C zugegeben und das Reaktionsgemisch 1 Std. bei -60 °C gehalten. Danach lässt man die Suspension innerhalb von 40 Min. auf 0 °C auftauen, hydrolisiert mit 100 ml 5N HCl und arbeitet auf. Man erhält 11,2 g (43 %) farbloses Oel vom Sdp. 121-124 °C/25 mbar.

$^1$H-NMR ($CDCl_3$); 60 MHz): 7,45 (d; J 2, 1H); 7,3 (m, 2H).

$^{13}$C-NMR ($CDCl_3$; 75,1 MHz): 181,7 (t; $J_{C\text{-}CF}$ 31,6; C=O); 133,3 (CH); 132,8 (-C< ); 132,4 (-C< ); 132,2 (CH); 131,5 (-C< ); 129,0 (CH); 119,5 (t; $J_{CF}$ 305,3; $CF_2Cl$).

Beispiel 22: Herstellung von α-Chlor-α,α-difluormethyl-(2,4,6-trichlorphenyl)-keton

In einem 750 ml Sulfierkolben werden bei -65 °C 230 ml (190 mmol) Methyllithium (0,82 M in DEE, 1 eq. LiBr enthaltend) während 30 Min. zu einer Suspension von 32,6 g (180 mmol) 1,3,5,-Trichlorbenzol in 180 ml THF getropft. Danach wird die orange Suspension innerhalb von 20 Min. auf -30 °C erwärmt und nachfolgend während 20 Min. bei -70 °C mit 42,6 g (190 mmol) α-Chlor-α,α-difluoracetyl-N-methylpiperazid, gelöst in 20 ml THF, versetzt. Man lässt die gelbe Suspension auf 0 °C auftauen und hydrolisiert mit 100 ml 5N HCl bei -20 °C. Man extrahiert die wässerige Phase 3-mal mit je 100 ml DEE. Die organischen Lösungen werden je 1-mal mit 50 ml 2N HCl und $H_2O$/Sole gewaschen, über $MgSO_4$ getrocknet und im VR eingeengt. Bei der Destillation des Rückstandes gehen beim Siedepunkt 70-71 °C/$10^{-2}$ mbar 45,7 g (86 %) des Produktes als farbloses Oel über.

$^1$H-NMR ($CDCl_3$; 300 MHz): 7,20 (s; 2H).

$^{13}$C-NMR ($CDCl_3$; 75,4 MHz): 183,5 (t; $J_{C\text{-}CF}$ 36,3; C=O); 138,0 (-C< ); 132,9 (2 x -C< ); 131,6 (-C< ); 128,4 (2 x CH); 119,4 (t; $J_{CF}$ 304,8; $CF_2Cl$).

Beispiel 23: Herstellung von α-Chlor-α,α-difluormethyl-(2,3,4-trichlorphenyl)-keton (A) und symmetrischen 1,3-Bis(α-chlor-α,α-difluoracetyl)-4,5,6-trichlorbenzol (B).

54,4 g (300 mmol) 1,2,3-Trichlorbenzol, gelöst in 100 ml THF, werden wie in Beispiel 20 bei -75 °C zu einer Suspension von 330 mmol Dicyclohexyllithiumamid [aus 60 g (330 mmol) Dicyclohexylamin und 205 ml (330 mmol) n-Butyllithium (1,61 M in Hexan; letzteres wird durch THF ersetzt)] in 200 ml THF getropft. Nach der Zugabe von 38,4 g (330 mmol) TMEDA in 100 ml THF bei -75 °C wird die rötliche, trübe Lösung 1 Std. bei -60 °C gerührt, dann für kurze Zeit auf -40 °C erwärmt. Anschliessend wird während 30 Min. eine Lösung von 64,0 g (320 mmol) α-Chlor-α,α-difluoracetylmorpholid in 100 ml THF bei -75 °C zugetropft, wobei sich eine braune Suspension bildet. Nach 30 minütigem Rühren bei -60 °C lässt man die Suspension innerhalb von 20 Min. auf 0 °C auftrauen und hydrolisiert unter $CO_2$/Ethanol-Kühlung mit 150 ml 5N HCl. Das Gemisch wird durch Hyflo filtriert und aufgearbeitet. Man erhält 76,3 g Produktgemisch, bestehend aus A und B im Verhältnis 7:1, die durch fraktionierte Destillation getrennt werden und als farblose Oele anfallen. Man erhält 57,3 g (65 %) A, Sdp. 80-82 °C/0,2 mbar und 8,4 g (7 %) B, Sdp. 87-89 °C/0,2 mbar.

$^1$H-NMR von A ($CDCl_3$; 300 MHz): 7,55 (d; J 8,0; 1H) 7,48 (d x t; J 8,0; 1,0; 1H).

$^{13}$C-NMR von A ($CDCl_3$; 75,4 MHz): 181,7 (t; $J_{C\text{-}CF}$ 31,8: C=O); 138,5 (-C< ); 133,1 (-C< ); 131,5 (-C< ); 134,1 (-C< ); 128,5 (CH); 126,7 (CH); 119,5 (t; $J_{CF}$ 305,4; $CF_2Cl$).

$^1$H-NMR von B ($CDCl_3$; 300 MHz): 7,48 (s; 2H).

## Ansprüche

1. Verfahren zur Herstellung von α-fluorierten Ketonen der Formel I

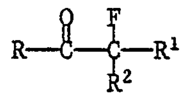 (I),

worin R lineares oder verzweigtes $C_1$-$C_{18}$-Alkyl-, $C_2$-$C_{18}$-Alkenyl $C_2$-$C_{18}$-Alkinyl; Cycloalkyl oder Cycloalkenyl mit 3 bis 8 Ring-C-Atomen, das mit $C_1$-$C_{12}$-Alkyl substituiert sein kann; über ein C-Atom an die CO-Gruppe gebundenes Heterocycloalkyl oder Heterocycloalkenyl mit 3 bis 7 Ringgliedern, das Heteroatome aus der Gruppe O, S und N enthält und das mit $C_1$-$C_{12}$-Alkyl substituiert sein kann; mit diesem Cycloalkyl, Cycloalkenyl, Heterocycloalkyl oder Heterocycloalkenyl substituiertes $C_1$-$C_{18}$-Alkyl, $C_2$-$C_{18}$-Alkenyl oder $C_2$-$C_{18}$-Alkinyl; $C_6$-$C_{14}$-Aryl, $C_7$-$C_{18}$-Alkaryl oder -Aralkyl, $C_8$-$C_{18}$-Alkaralkyl, -Aralkenyl oder -Aralkinyl, $C_9$-$C_{18}$-Alkaralkenyl oder -Alkaralkinyl; Heteroaryl mit 5 oder 6 Ringatomen, das Heteroatome aus der Gruppe O, N und S enthält und das mit $C_1$-$C_{12}$-Alkyl substituiert sein kann; oder mit diesem Heteroaryl substituiertes $C_1$-$C_{18}$-Alkyl, $C_2$-$C_{18}$-Alkenyl, $C_2$-$C_{18}$-Alkinyl darstellt; wobei dieser Rest R unsubstituiert oder mit Phenyl, Phenyl-$C_1$-$C_4$-alkyl, Phenoxy, Phenylthio, Benzyloxy, Benzylthio, $C_5$- oder $C_6$-Cycloalkyl, $C_5$- oder $C_6$-Cycloalkoxy, $C_5$-oder $C_6$-Cycloalkylthio, $C_1$-$C_{12}$-Alkoxy, $C_1$-$C_{12}$-Alkylthio, -Halogen, $-NO_2$, Sekundäramino, $C_1$-$C_{12}$-Acyl, $C_1$-$C_{12}$-Acylamino, $C_1$-$C_{12}$-Aminocarbonyl, $R^3_3$ SiO-, wobei $R^3$ Phenyl, Benzyl oder $C_1$-$C_8$-Alkyl bedeutet, -O-$C_1$-$C_4$-Alkylen-O- oder -S-$C_1$-$C_4$-Alkylen-S- substituiert ist, $R^1$ und $R^2$ unabhängig voneinander für H, Halogen $C_1$-$C_{18}$-Alkyl, Cycloalkyl oder Cycloalkenyl mit 3 bis 8 Ringkohlenstoffatomen, $C_6$-$C_{14}$-Aryl, $C_7$-$C_{18}$-Alkaryl oder -Aralkyl, $C_8$-$C_{18}$-Aralkenyl, -Aralkinyl oder -Alkaralkyl, $C_9$-$C_{18}$-Alkaralkenyl oder -Alkaralkinyl, $C_2$-$C_{18}$-Alkenyl, $C_2$-$C_{18}$-Alkinyl, Heterocycloalkyl oder Heterocycloalkenyl mit 3 bis 7 Ringgliedern, oder Heteroaryl mit 5 oder 6 Ringgliedern und Heteroatomen aus der Gruppe N, O und S; mit dem genannten Cycloalkyl, Heterocycloalkyl, Heterocycloalkenyl oder Heteroaryl substituiertes $C_1$-$C_{18}$-Alkyl, $C_2$-$C_{18}$-Alkenyl oder $C_2$-$C_{18}$- Alkinyl, wobei $R^1$ und $R^2$ unsubstituiert oder wie für R definiert substituiert sein können, dadurch gekennzeichnet, dass man in einem inerten Lösungsmittel ein Carbonsäureamid der Formel II

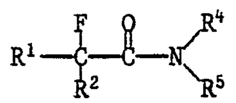 (II),

worin $R^1$ und $R^2$ die angegebene Bedeutung haben, $R^4$ $C_1$-$C_6$-Alkyl-X-$C_nH_{2n}$-ist, worin X für -O- oder -N($C_1$-$C_{12}$-Alkyl)- und n für eine Zahl von 2 bis 4 stehen, $R^5$ $C_1$-$C_{12}$-Alkyl oder $C_1$-$C_{12}$-Alkyl-X-$C_nH_{2n}$-darstellt, oder $R^4$ und $R^5$ zusammen mit dem N-Atom, an das sie gebunden sind, einen gesättigten oder ungesättigten Heterocyclus mit 5 bis 7 Ringgliedern darstellen, der insgesamt mindestens 2 Heteroatome aus der Gruppe O, S und $NR^6$ enthält und unsubstituiert oder mit $C_1$-$C_6$-Alkyl, substituiert ist, und wobei $R^6$ für $C_1$-$C_6$-Alkyl, Phenyl, $C_1$-$C_{12}$-Alkylphenyl, Benzyl oder $C_1$-$C_{12}$-Alkylbenzyl steht, mit einer Verbindung der Formel III

$R$-$M^xY_{(x-1)}$ (III),

worin R die zuvor angegebene Bedeutung hat, Y für Halogenid, $M^x$ für ein Metallion aus der Gruppe $Li^{\oplus}$, $Na^{\oplus}$, $K^{\oplus}$, $Rb^{\oplus}$, $Mg^{2\oplus}$, $Zn^{2\oplus}$, $Cd^{2\oplus}$, $Cu^{\oplus}$, $Cu^{2\oplus}$,$Al^{3\oplus}$, $Ti^{4\oplus}$ und $Sn^{4\oplus}$ stehen und x die Valenz des Metallions bedeutet, zu einer Verbindung der Formel IV

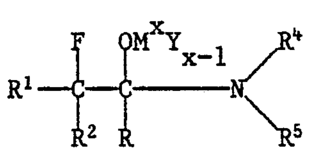 (IV),

worin R, $R^1$, $R^2$, $R^4$, $R^5$, $M^x$, Y und x die zuvor angegebene Bedeutung haben, umsetzt, und die Verbindung der Formel IV zu einer Verbindung der Formel I hydrolysiert.

2. Verfahren gemäss Anspruch 1, dadurch gekennzeichnet, dass R, $R^1$ oder $R^2$ unsubstituiert oder mit Phenyl, Benzyl, Phenoxy, Benzyloxy, $C_1$-$C_{12}$-Alkoxy, $C_1$-$C_{12}$-Alkylthio, F, Cl, Sekundäramino, oder $R^3_3$ SiO substituiert sind.

3. Verfahren gemäss Anspruch 1, dadurch gekennzeichnet, dass R lineares oder verzweigtes $C_1$-$C_{12}$-Alkyl, $C_2$-$C_{12}$-Alkenyl, $C_2$-$C_{12}$-Alkinyl, Cycloalkyl oder Cycloalkenyl mit 5 oder 6 Ring-C-Atomen, Heterocycloalkyl mit 5 oder 6 Ringgliedern, $C_6$-$C_{14}$-Aryl, $C_7$-$C_{12}$-Alkaryl oder -Aralkyl, $C_8$-$C_{14}$-Alkaralkyl, -Aralkenyl oder -Aralkinyl, $C_9$-$C_{16}$-Alkaralkenyl oder -Alkaralkinyl, oder Heteroaryl mit 5 oder 6 Ringgliedern darstellt.

4. Verfahren gemäss Anspruch 1, dadurch gekennzeichnet, dass $R^1$ und $R^2$ unabhängig voneinander für H, F, Cl, Br, -$C_1$-$C_{12}$-Alkyl, Cycloalkyl mit 5 oder 6 Ring-C-Atomen, $C_6$-$C_{12}$-Aryl, $C_7$-$C_{14}$-Aralkyl oder -Alkaryl, $C_8$-$C_{14}$-Alkaralkyl, -Aralkenyl oder -Aralkinyl, $C_9$-$C_{14}$-Alkaralkenyl oder -Alkaralkinyl, $C_2$-$C_{12}$-Alkenyl, $C_2$-$C_{12}$-Alkinyl, Heterocycloalkyl, Heterocycloalkenyl oder Heteroaryl mit 5 oder 6 Ringgliedern und ein oder zwei Heteroatomen aus der Gruppe N, O und S stehen.

5. Verfahren gemäss Anspruch 1, dadurch gekennzeichnet, dass $R^1$ für H, F, Cl oder Br steht und $R^2$ die in Anspruch 1 angegebene Bedeutung hat.

6. Verfahren gemäss Anspruch 5, dadurch gekennzeichnet, das $R^1$ für F steht.

7. Verfahren gemäss Anspruch 5, dadurch gekennzeichnet, dass $R^2$ für H, F, Cl, Br, $C_1$-$C_6$-Perfluoralkyl, $C_1$-$C_{12}$-Alkyl, $C_1$-$C_{12}$-Alkinyl, Cycloalkyl mit 5 oder 6 Ring-C-Atomen, $C_6$-$C_{12}$-Aryl, $C_7$-$C_{17}$-Aralkyl oder -Alkaryl oder $C_8$-$C_{14}$-Alkaralkyl steht.

8. Verfahren gemäss Anspruch 5, dadurch gekennzeichnet, dass $R^1$ für F steht und $R^2$ H, F, Cl, Br, $C_1$-$C_4$-Perfluoralkyl, $C_1$-$C_6$-Alkyl oder -Alkenyl bedeutet.

9. Verfahren gemäss Anspruch 1, dadurch gekennzeichnet, dass $R^4$ $C_1$-$C_4$-Alkyl-X-$C_nH_{2n}$- ist, worin X -O- oder -N($C_1$-$C_4$-Alkyl)- und n 2 oder 3 sind und $R^5$ diese Bedeutung von $R^4$ hat oder $C_1$-$C_4$-Alkyl darstellt.

10. Verfahren gemäss Anspruch 1, dadurch gekennzeichnet, dass $R^4$ und $R^5$ zusammen mit dem N-Atom, an das sie gebunden sind, den Rest eines 5-oder 6-gliedrigen, gesättigten oder ungesättigten Heterocyclus mit 1 oder 2 weiteren Heteroatomen aus der Gruppe O, S und $NR^6$ darstellen, und $R^6$ die im Anspruch 1 angegebene Bedeutung hat.

11. Verfahren gemäss Anspruch 10, dadurch gekennzeichnet, dass $R^6$ für $C_1$-$C_4$-Alkyl oder Phenyl steht.

12. Verfahren gemäss Anspruch 10, dadurch gekennzeichnet, dass $R^4$ und $R^5$ zusammen mit dem N-Atom einen Rest der Formeln

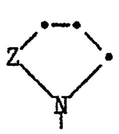 , 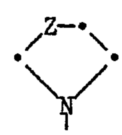 , 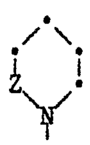 , 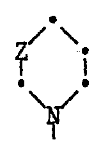 oder 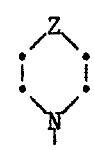

darstellen, worin Z für O, S oder $NR^6$ steht und $R^6$ $C_1$-$C_4$-Alkyl oder Phenyl bedeutet.

13. Verfahren gemäss Anspruch 12, dadurch gekennzeichnet, dass $R^4$ und $R^5$ zusammen mit dem N-Atom einen Rest der Formeln

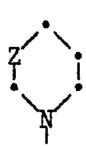 oder 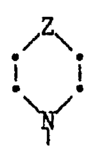

darstellen, worin Z O, S oder $NCH_3$ bedeutet.

14. Verfahren gemäss Anspruch 1, dadurch gekennzeichnet, dass Y in Formel III für Cl oder Br steht.

15. Verfahren gemäss Anspruch 1, dadurch gekennzeichnet, dass $M^x$ für $Li^{\oplus}$, $Mg^{2\oplus}$ oder $Zn^{2\oplus}$ steht.

16. Verfahren gemäss Anspruch 1, dadurch gekennzeichnet, dass die Reaktionstemperatur -80 °C bis 60 °C beträgt.

17. Verfahren gemäss Anspruch 16, dadurch gekennzeichnet, dass das inerte Lösungsmittel ein aliphatischer oder cycloaliphatischer Ether ist.

18. Verfahren gemäss Anspruch 1, dadurch gekennzeichnet, dass die kristalline Verbindung der Formel IV vor der Hydrolyse isoliert wird.

19. Verbindungen der Formel IV

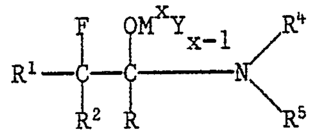 (IV),

worin $R^1$, $R^2$, $R^4$, $R^5$, M, Y und x die in Anspruch 1 angegebene Bedeutung haben.

20. Verbindungen der Formel Ia

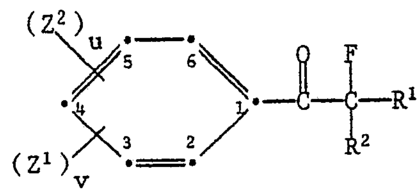 (Ia),

worin $R^1$ F oder Cl bedeutet und $R^2$ H ist oder unabhängig die Bedeutung von $R^1$ hat, $Z^1$ Cl und $Z^2$ F darstellen, v eine Zahl von 1 bis 5 und u 0 oder eine Zahl von 1 bis 4 sind, und die Summe u + v 2 bis 5 ist, ausgenommen 1-(2,4-Dichlorphenyl)-2,2,2-trifluorethanon.

Patentansprüche: für den folgenden Vertragsstaat: ES

1. Verfahren zur Herstellung von α-fluorierten Ketonen der Formel I

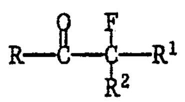 (I),

worin R lineares oder verzweigtes $C_1$-$C_{18}$-Alkyl-, $C_2$-$C_{18}$-Alkenyl $C_2$-$C_{18}$-Alkinyl; Cycloalkyl oder Cycloalkenyl mit 3 bis 8 Ring-C-Atomen, das mit $C_1$-$C_{12}$-Alkyl substituiert sein kann; über ein C-Atom an die CO-Gruppe gebundenes Heterocycloalkyl oder Heterocycloalkenyl mit 3 bis 7 Ringgliedern, das Heteroatome aus der Gruppe O, S und N enthält und das mit $C_1$-$C_{12}$-Alkyl substituiert sein kann; mit diesem Cycloalkyl, Cycloalkenyl, Heterocycloalkyl oder Heterocycloalkenyl substituiertes $C_1$-$C_{18}$-Alkyl, $C_2$-$C_{18}$-Alkenyl oder $C_2$-$C_{18}$-Alkinyl; $C_6$-$C_{14}$-Aryl, $C_7$-$C_{18}$-Alkaryl oder -Aralkyl, $C_8$-$C_{18}$-Alkaralkyl, -Aralkenyl oder -Aralkinyl, $C_9$-$C_{18}$-Alkaralkenyl oder -Alkaralkinyl; Heteroaryl mit 5 oder 6 Ringatomen, das Heteroatome aus der Gruppe O, N und S enthält und das mit $C_1$-$C_{12}$-Alkyl substituiert sein kann; oder mit diesem Heteroaryl substituiertes $C_1$-$C_{18}$-Alkyl, $C_2$-$C_{18}$-Alkenyl, $C_2$-$C_{18}$-Alkinyl darstellt; wobei dieser Rest R unsubstituiert oder mit Phenyl, Phenyl-$C_1$-$C_4$-alkyl, Phenoxy, Phenylthio, Benzyloxy, Benzylthio, $C_5$- oder $C_6$-Cycloalkyl, $C_5$- oder $C_6$-Cycloalkoxy, $C_5$-oder $C_6$-Cycloalkylthio, $C_1$-$C_{12}$-Alkoxy, $C_1$-$C_{12}$-Alkylthio, -Halogen, -$NO_2$, Sekundäramino, $C_1$-$C_{12}$-Acyl, $C_1$-$C_{12}$-Acylamino, $C_1$-$C_{12}$-Aminocarbonyl, $R^3_3$ SiO-, wobei $R^3$ Phenyl, Benzyl oder $C_1$-$C_8$-Alkyl bedeutet, -O-$C_1$-$C_4$-Alkylen-O- oder -S-$C_1$-$C_4$-Alkylen-S- substituiert ist, $R^1$ und $R^2$ unabhängig voneinander für H, Halogen $C_1$-$C_{18}$-Alkyl, Cycloalkyl oder Cycloalkenyl mit 3 bis 8 Ringkohlenstoffatomen, $C_6$-$C_{14}$-Aryl, $C_7$-$C_{18}$-Alkaryl oder -Aralkyl, $C_8$-$C_{18}$-Aralkenyl, -Aralkinyl oder -Alkaralkyl, $C_9$-$C_{18}$-Alkaralkenyl oder -Alkaralkinyl, $C_2$-$C_{18}$-Alkenyl, $C_2$-$C_{18}$-Alkinyl, Heterocycloalkyl oder Heterocycloalkenyl mit 3 bis 7 Ringgliedern, oder Heteroaryl mit 5 oder 6 Ringgliedern und Heteroatomen aus der Gruppe N, O und S; mit dem genannten Cycloalkyl, Heterocycloalkyl, Heterocycloalkenyl oder Heteroaryl substituiertes $C_1$-$C_{18}$-Alkyl, $C_2$-$C_{18}$-Alkenyl oder $C_2$-$C_{18}$- Alkinyl, wobei $R^1$ und $R^2$ unsubstituiert oder wie für R definiert substituiert sein können, dadurch gekennzeichnet, dass man in einem inerten Lösungsmittel ein Carbonsäureamid der Formel II

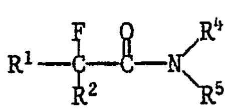 (II),

worin $R^1$ und $R^2$ die angegebene Bedeutung haben, $R^4$ $C_1$-$C_6$-Alkyl-X-$C_nH_{2n}$-ist, worin X für -O- oder -N($C_1$-$C_{12}$-Alkyl)- und n für eine Zahl von 2 bis 4 stehen, $R^5$ $C_1$-$C_{12}$-Alkyl oder $C_1$-$C_{12}$-Alkyl-X-$C_nH_{2n}$-darstellt,

oder $R^4$ und $R^5$ zusammen mit dem N-Atom, an das sie gebunden sind, einen gesättigten oder ungesättigten Heterocyclus mit 5 bis 7 Ringgliedern darstellen, der insgesamt mindestens 2 Heteroatome aus der Gruppe O, S und $NR^6$ enthält und unsubstituiert oder mit $C_1$-$C_6$-Alkyl, substituiert ist, und wobei $R^6$ für $C_1$-$C_6$-Alkyl, Phenyl, $C_1$-$C_{12}$-Alkylphenyl, Benzyl oder $C_1$-$C_{12}$-Alkylbenzyl steht, mit einer Verbindung der Formel III

$R$-$M^xY_{(x-1)}$ (III), worin R die zuvor angegebene Bedeutung hat, Y für Halogenid, $M^x$ für ein Metallion aus der Gruppe $Li^{\oplus}$, $Na^{\oplus}$, $K^{\oplus}$, $Rb^{\oplus}$, $Mg^{2\oplus}$, $Zn^{2\oplus}$, $Cd^{2\oplus}$, $Cu^{\oplus}$, $Cu^{2\oplus}$, $Al^{3\oplus}$, $Ti^{4\oplus}$ und $Sn^{4\oplus}$ stehen und x die Valenz des Metallions bedeutet, zu einer Verbindung der Formel IV

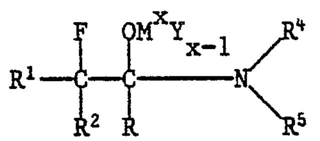

(IV),

worin R, $R^1$, $R^2$, $R^4$, $R^5$, $M^x$, Y und x die zuvor angegebene Bedeutung haben, umsetzt, und die Verbindung der Formel IV zu einer Verbindung der Formel I hydrolysiert.

2. Verfahren gemäss Anspruch 1, dadurch gekennzeichnet, dass R, $R^1$ oder $R^2$ unsubstituiert oder mit Phenyl, Benzyl, Phenoxy, Benzyloxy, $C_1$-$C_{12}$-Alkoxy, $C_1$-$C_{12}$-Alkylthio, F, Cl, Sekundäramino, oder $R^3_3$ SiO substituiert sind.

3. Verfahren gemäss Anspruch 1, dadurch gekennzeichnet, dass R lineares oder verzweigtes $C_1$-$C_{12}$-Alkyl, $C_2$-$C_{12}$-Alkenyl, $C_2$-$C_{12}$-Alkinyl, Cycloalkyl oder Cycloalkenyl mit 5 oder 6 Ring-C-Atomen, Heterocycloalkyl mit 5 oder 6 Ringgliedern, $C_6$-$C_{14}$-Aryl, $C_7$-$C_{12}$-Alkaryl oder -Aralkyl, $C_8$-$C_{14}$-Alkaralkyl, -Aralkenyl oder -Aralkinyl, $C_9$-$C_{16}$-Alkaralkenyl oder -Alkaralkinyl, oder Heteroaryl mit 5 oder 6 Ringgliedern darstellt.

4. Verfahren gemäss Anspruch 1, dadurch gekennzeichnet, dass $R^1$ und $R^2$ unabhängig voneinander für H, F, Cl, Br, $C_1$-$C_{12}$-Alkyl, Cycloalkyl mit 5 oder 6 Ring-C-Atomen, $C_6$-$C_{12}$-Aryl, $C_7$-$C_{14}$-Aralkyl oder -Alkaryl, $C_8$-$C_{14}$-Alkaralkyl, -Aralkenyl oder -Aralkinyl, $C_9$-$C_{14}$-Alkaralkenyl oder -Alkaralkinyl, $C_2$-$C_{12}$-Alkenyl, $C_2$-$C_{12}$-Alkinyl, Heterocycloalkyl, Heterocycloalkenyl oder Heteroaryl mit 5 oder 6 Ringgliedern und ein oder zwei Heteroatomen aus der Gruppe N, O und S stehen.

5. Verfahren gemäss Anspruch 1, dadurch gekennzeichnet, dass $R^1$ für H, F, Cl oder Br steht und $R^2$ die in Anspruch 1 angegebene Bedeutung hat.

6.Verfahren gemäss Anspruch 5, dadurch gekennzeichnet, das $R^1$ für steht.

7. Verfahren gemäss Anspruch 5, dadurch gekennzeichnet, dass $R^2$ für H, F, Cl, Br, $C_1$-$C_6$-Perfluoralkyl, $C_1$-$C_{12}$-Alkyl, $C_1$-$C_{12}$-Alkinyl, Cycloalkyl mit 5 oder 6 Ring-C-Atomen, $C_6$-$C_{12}$-Aryl, $C_7$-$C_{17}$-Aralkyl oder -Alkaryl oder $C_8$-$C_{14}$-Alkaralkyl steht.

8. Verfahren gemäss Anspruch 5, dadurch gekennzeichnet, dass $R^1$ für F steht und $R^2$ H, F, Cl, Br, $C_1$-$C_4$-Perfluoralkyl, $C_1$-$C_6$-Alkyl oder -Alkenyl bedeutet.

9. Verfahren gemäss Anspruch 1, dadurch gekennzeichnet, dass $R^4$ $C_1$-$C_4$-Alkyl-X-$C_nH_{2n}$- ist, worin X -O- oder -N($C_1$-$C_4$-Alkyl)- und n 2 oder 3 sind und $R^5$ diese Bedeutung von $R^4$ hat oder $C_1$-$C_4$-Alkyl darstellt.

10. Verfahren gemäss Anspruch 1, dadurch gekennzeichnet, dass $R^4$ und $R^5$ zusammen mit dem N-Atom, an das sie gebunden sind, den Rest eines 5-oder 6-gliedrigen, gesättigten oder ungesättigten Heterocyclus mit 1 oder 2 weiteren Heteroatomen aus der Gruppe O, S und $NR^6$ darstellen, und $R^6$ die im Anspruch 1 angegebene Bedeutung hat.

11. Verfahren gemäss Anspruch 10, dadurch gekennzeichnet, dass $R^6$ für $C_1$-$C_4$-Alkyl oder Phenyl steht.

12. Verfahren gemäss Anspruch 10, dadurch gekennzeichnet, dass $R^4$ und $R^5$ zusammen mit dem N-Atom einen Rest der Formeln

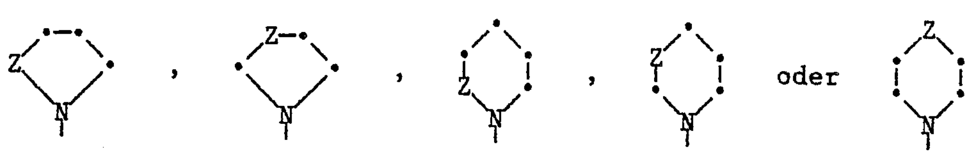

darstellen, worin Z für O, S oder $NR^6$ steht und $R^6$ $C_1$-$C_4$-Alkyl oder Phenyl bedeutet.

13. Verfahren gemäss Anspruch 12, dadurch gekennzeichnet, dass $R^4$ und $R^5$ zusammen mit dem N-Atom einen Rest der Formeln

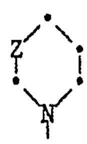 oder 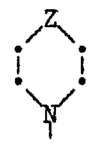

darstellen, worin Z O, S oder $NCH_3$ bedeutet.

14. Verfahren gemäss Anspruch 1, dadurch gekennzeichnet, dass Y in Formel III für Cl oder Br steht.

15. Verfahren gemäss Anspruch 1, dadurch gekennzeichnet, dass $M^x$ für $Li^{\oplus}$, $Mg^{2\oplus}$ oder $Zn^{2\oplus}$ steht.

16. Verfahren gemäss Anspruch 1, dadurch gekennzeichnet, dass die Reaktionstemperatur -80 °C bis 60 °C beträgt.

17. Verfahren gemäss Anspruch 16, dadurch gekennzeichnet, dass das inerte Lösungsmittel ein aliphatischer oder cycloaliphatischer Ether ist.

18. Verfahren gemäss Anspruch 1, dadurch gekennzeichnet, dass die kristalline Verbindung der Formel IV vor der Hydrolyse isoliert wird.

19. Verfahren gemäss Anspruch 1, dadurch gekennzeichnet, dass man

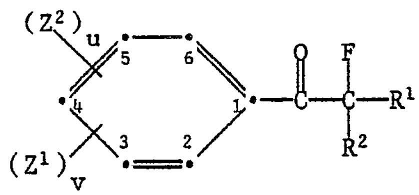 (Ia),

worin $R^1$ F oder Cl bedeutet und $R^2$ H ist oder unabhängig die Bedeutung von $R^1$ hat, $Z^1$ Cl und $Z^2$ F darstellen, v eine Zahl von 1 bis 5 und u 0 oder eine Zahl von 1 bis 4 sind, und die Summe u + v 2 bis 5 ist, ausgenommen 1-(2,4-Dichlorphenyl)-2,2,2-trifluorethanon, herstellt.